# EUROPEAN PATENT APPLICATION

(11) **EP 4 014 861 A1**
(43) Date of publication of application: **22.06.2022**
(21) Application number: 21150936.9
(22) Date of filing: 11.01.2021
(51) Int. Cl.: A61B 5/083, A61B 5/145, A61B 5/00, G16H 50/20, G16H 50/30

(54) **GRAPHICAL REPRESENTATION OF OXYGENATION**

(30) Priority: 16.12.2020 US 202063126016 P
(71) Applicant: Koninklijke Philips N.V., 5656 AG Eindhoven (NL)
(72) Inventor: KACZMAREK, Piotr, 5656 AE Eindhoven (NL)
(74) Representative: Philips Intellectual Property & Standards

(57) **Abstract**

Methods and apparatus disclosed herein relate to graphical representation of oxygenation of an organism. In various embodiments, one or more measured physiological parameters of an organism may be analyzed. Based on the analysis, a graphical user interface (GUI) may be rendered. The GUI may convey oxygenation of an organism visually as a continuous physiological flow graphical element (236) that passes through four quadrants in a cycle. In various embodiments, each quadrant includes an abscissa and an ordinate that represent a relationship between at least two of the measured physiological parameters. In various embodiments, for at least some of the four quadrants, the ordinate of one quadrant comprises the abscissa of the next quadrant of the cycle.

## Description

### TECHNICAL FIELD

Various embodiments described herein are directed generally to health care. More particularly, but not exclusively, various methods and apparatus disclosed herein relate to graphical representation of the process of oxygen delivery (or "oxygenation") to an organism.

### BACKGROUND

Large numbers of physiological parameters may be measured for an organism, especially if they are in a heighted state of monitoring, such as being in an intensive care unit (ICU). Presenting these measured physiological parameters to clinicians such as doctors, nurses, veterinarians, *etc.,* can be challenging. Presenting too many measured physiological parameters can be overwhelming, while presenting too few may not sufficiently inform the clinician of how oxygenation of the organism is progressing.

In the patient monitoring context, many existing patient monitors are devoted to representing numerical displays and waveforms as part of a graphical user interface (GUI). However, existing oxygenation system GUIs suffer from various issues. Some offer too much detail and/or are too complex, straining a clinician's cognitive abilities. Others may offer too little detail, which may fail to provide timely warning of a deterioration in a condition of an organism and/or an efficacy of oxygen delivery therapy being applied to the organism. Currently, vital parameters are commonly displayed as set of numbers or trends along a timeline. However, clinical decisions are more often based upon concepts that are derived by combining parameter relationships and additional contextual information.

### SUMMARY

The present disclosure is directed to methods and apparatus for graphical representation of oxygenation of organisms. For example, in various embodiments, a GUI that is rendered on a display may include one or more graphical elements that convey, in a manner that is intuitive and quickly-digestible, various measured physiological parameters associated with oxygenation of an organism and the relationships between those parameters and the oxygenation process. In various embodiments, these graphical elements may have various spatial dimensions that are selected to convey physiological parameters associated with oxygenation, and/or relationships between those parameters.

For example, a GUI configured with selected aspects of the present disclosure may include what will be referred to herein as a "continuous physiological flow" graphical element that conveys relationships between various physiological parameters of an organism. In various embodiments, this continuous flow graphical element may be rendered on a display so that it passes (*e.g.*, cycles) through some number of visually-annotated regions, such as four quadrants, that represent various aspects and/or stages of the oxygenation process. These various aspects of the oxygenation process may include, for instance, lung function, gas transfer, blood/hemoglobin, and cardiac output. In each region, or quadrant, various visual aspects of the graphical element may be selected in order to convey information about various physiological parameters of the organism. For example, in many embodiments, the continuous physiological flow graphical element may be rendered as a path or arrow that passes through four quadrants in a clockwise or counter-clockwise direction. In each quadrant, a thickness or other spatial aspect of the continuous physiological flow graphical element may be selected to convey one or more physiological parameters.

An "organism" may include any living thing such as a human or animal that relies on oxygen in its breathing process, such as a human patient in a health care setting such as a hospital. The process of obtaining oxygen and providing it to various components of an organism is referred to as "oxygenation." With regard to the continuous physiological flow graphical element, the word "continuous" is meant to convey the continuous nature of the physiological process represented by the graphical element, and in particular, how values of various physiological parameters of an organism influence other physiological parameters. The graphical element itself may not necessarily be unbroken visually throughout its entirety in all implementations, although it may be unbroken and continuous visually in many implementations.

In some embodiments, the graphical element may be interactive, and may allow a clinician to adjust various values. Such an interactive version of the graphical element may be used as a simple, physiology-based model for a clinician to estimate the consequences of change in one or more physiological parameters or other parameters on the final outcome of the oxygenation process. For example, a clinician may be able to interact with the graphical element to determine by how much hemoglobin concentration should be to be increased in order to achieve acceptable level of oxygenation in observed patient under current conditions.

Embodiments described herein give rise to various technical advantages. The continuous physiological flow graphical element's representation of multiple physiological parameters and the relationships between them and the oxygenation process allows medical personnel to quickly understand the progress, efficacy, and/or state of an organism's oxygenation in real time. Moreover, in embodiments where components of the continuous physiological flow graphical element are given relative thicknesses, a clinician can quickly ascertain where problems may be originating and/or what other areas those problems may affect, and/ may identify a malady that causes the problems and/or a treatment, thereby improving the quality assurance of a clinical decision derived from reading these physiological parameters. For example, normalizing different measured physiological parameters in order that they be conveyed or "encoded" into various points of one or more continuous shapes (*e.g.,* the continuous physiological flow graphical element) may provide clinician's with a clear idea of what's "normal" or "baseline," and how (and/or why) a particular organism's oxygenation process diverges from a normal/baseline/expected oxygenation process.

Moreover, given their compact nature, it is possible to render continuous physiological flow graphical elements in a small area, such as a smart watch display, a patient monitor (which typically have small displays), and/or a small region of a nurse station's monitor (to allow information for other patients to also be displayed at the same time), and yet a large amount of information is still conveyed. This also allows for numerous physiological parameters to be conveyed in a condensed space, such that multiple continuous physiological flow graphical elements can be display on a screen at once, avoiding the need for scrolling or switching menus, thereby improving human-machine interaction. In some implementations, continuous physiological flow graphical elements may even be rendered in a paper or electronic document, such as an EMR, as a snapshot of the organism's oxygenation process.

Generally, in one aspect, a method may be implemented using one or more processors, and may include: analyzing one or more physiological parameters of an organism; based on the analyzing, causing a graphical user interface (GUI) to be rendered on a display, wherein the graphical user interface conveys oxygenation of an organism visually as a continuous physiological flow graphical element that passes through four quadrants in a cycle, wherein the causing includes: causing each of the quadrants to include an abscissa and an ordinate that represent a relationship between at least two of the physiological parameters; and for at least some of the four quadrants, causing the ordinate of one quadrant to correspond to the abscissa of the next quadrant of the cycle.

In various embodiments, in a first quadrant of the four quadrants that conveys information about lung function in the organism, the abscissa may correspond to fractional oxygen concentration (FiO2) of the organism and the ordinate may correspond to partial pressure of arterial oxygen (PaO2) of the organism. In various embodiments, in a second quadrant of the four quadrants that conveys information about gas transfer in the organism, the abscissa may correspond to PaO2 of the organism and the ordinate may correspond to hemoglobin oxygen saturation (SaO2) of the organism.

In various embodiments, the method may include visually conveying the relationship between PaO2 values of the abscissa and SaO2 value of the ordinate by causing an oxygen-hemoglobin dissociation curve to be rendered in the second quadrant. In various embodiments, the method may include rendering one or more additional visual annotations that convey one or more additional parameters that influence a portion of the oxygen-hemoglobin dissociation curve.

In various embodiments, in a third quadrant of the four quadrants that conveys information about blood of the organism, the abscissa may correspond to SaO2 of the organism and the ordinate may correspond to arterial oxygen content (CaO2) of the organism. In various embodiments, a width of the continuous physiological flow graphical element that passes from the second quadrant to the third quadrant is dictated by an intersection of the continuous physiological flow graphical element with the oxygen-hemoglobin dissociation curve. In various embodiments, in a fourth quadrant of the four quadrants that conveys information about cardiac output of the organism, the abscissa may correspond to CaO2 of the organism and the ordinate may correspond to oxygen delivery (DO2) of the organism.

In various embodiments, the method may include, for each of the four quadrants, causing the continuous physiological flow graphical element to have a thickness that corresponds to a physiological value of the organism on the respective abscissa of the quadrant.

In addition, some implementations include one or more processors of one or more computing devices, where the one or more processors are operable to execute instructions stored in associated memory, and where the instructions are configured to cause performance of any of the aforementioned methods. Some implementations also include one or more non-transitory computer readable storage media storing computer instructions executable by one or more processors to perform any of the aforementioned methods.

It should be appreciated that all combinations of the foregoing concepts and additional concepts discussed in greater detail below (provided such concepts are not mutually inconsistent) are contemplated as being part of the inventive organism matter disclosed herein. In particular, all combinations of claimed organism matter appearing at the end of this disclosure are contemplated as being part of the inventive organism matter disclosed herein. It should also be appreciated that terminology explicitly employed herein that also may appear in any disclosure incorporated by reference should be accorded a meaning most consistent with the particular concepts disclosed herein.

### BRIEF DESCRIPTION OF THE DRAWINGS

In the drawings, like reference characters generally refer to the same parts throughout the different views. Also, the drawings are not necessarily to scale, emphasis instead generally being placed upon illustrating various principles of the embodiments described herein.
Fig. 1 illustrates an example environment in which selected aspects of the present disclosure may be practiced.
Figs. 2A, 2B, 2C, and 2D depict examples of how individual quadrants of a GUI rendered in accordance with aspects of the present disclosure may correspond to various aspects and/or stages of an oxygenation process, in accordance with selected aspects of the present disclosure.
Figs. 3A and 3B demonstrate examples of how the quadrants of Figs. 2A-D may be arranged visually relative to one another, in accordance with various embodiments.
Fig. 4 depicts an example GUI configured with selected aspects of the present disclosure.
Fig. 5 depicts another example GUI configured with selected aspects of the present disclosure.
Fig. 6 depicts another example GUI configured with selected aspects of the present disclosure.
Fig. 7 depicts another example GUI configured with selected aspects of the present disclosure.
Fig. 8 depicts another example GUI configured with selected aspects of the present disclosure.
Fig. 9 depicts an example method for practicing selected aspects of the present disclosure.
Fig. 10 depicts an example computing system architecture.

### DETAILED DESCRIPTION

Large numbers of physiological parameters may be measured for an organism, especially if they are in a heighted state of monitoring, such as being in an intensive care unit (ICU). Mental processing of these measured physiological parameters by health care personnel such as doctors and nurses can strain their cognitive abilities. For example, the information presented by many existing oxygenation GUIs may not be effective in demonstrating how various physiological parameters associated with oxygen delivery to an organism interact with each other. In view of the foregoing, various embodiments and implementations of the present disclosure are directed to improved graphical representation of oxygenation of an organism.

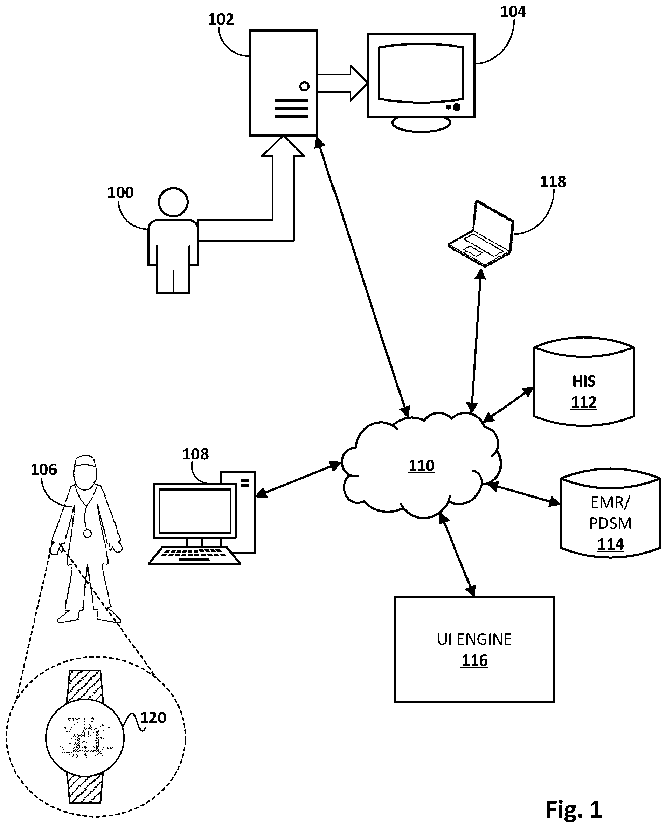

Fig. 1 depicts an example environment with various components that may practice selected aspects of the present disclosure. An organism such as a patient 100 may have various physiological parameters measured, *e.g.,* by various types of probes, electrodes, laboratory tests, swabs, *etc.* One or more of those measured physiological parameters may be provided to a local computing device 102 that is operably coupled with a patient monitor 104. In some embodiments, patient monitor 104 may be a standalone computing device with onboard logic such as processor(s) and memory, in which case computing device 102 may be omitted. Patient monitor 104 may display a graphical user interface ("GUI", not depicted in Fig. 1) that is configured with selected aspects of the present disclosure to convey information about progression of an oxygenation process undergone by patient 100. In some embodiments, the oxygenation process may involve providing that patient 100 with supplemental oxygen. Additionally or alternatively, in some embodiments, the oxygenation process may be unassisted, *i.e.* patient 100 may obtain oxygen naturally from the air.

Health care personnel 106 such as doctors, nurses, clinicians, *etc.,* may read patient monitor 104 directly, and/or may operate various types of client computing devices 108 to view GUIs rendered with selected aspects of the present disclosure. Computing device 108 (or other computing devices mentioned herein) may take various forms, such as one or more of: a desktop computing device, a laptop computing device, a tablet computing device, a mobile phone computing device, a computing device of a vehicle of the user (*e.g.,* an in-vehicle communications system, an in-vehicle entertainment system, an in-vehicle navigation system), a standalone interactive speaker (which in some cases may include a vision sensor), a smart appliance such as a smart television (or a standard television equipped with a networked dongle with automated assistant capabilities), and/or a wearable apparatus of the user that includes a computing device (*e.g.,* a watch of the user having a computing device, glasses of the user having a computing device, a virtual or augmented reality computing device). Additional and/or alternative client computing devices may be provided.

Various components depicted in Fig. 1, such as computing device 102, computing device 108, and/or patient monitor 104, may be in network communication with each other over one or more computer networks 110. One or more computer networks 110 may include wired and/or wireless local area and/or wide area networks (*e.g.,* the Internet). Various computer networking technologies may be implemented to facilitate communication between the various components, such as Wi-Fi, cellular, Ethernet, fiber optic, *etc.*

Various information systems may be provided that are accessible to obtain various data points relevant to the present disclosure. For example, a hospital information system (HIS) 112 and/or an EMR/PDMS system 114 may receive and/or maintain, e.g., as part of EMRs for organisms, measured physiological parameters. A user interface (UI) engine 116 may be configured to practice selected aspects of the present disclosure in order to cause various display devices, such as patient monitor 104, computing device 108, or other devices such as a laptop computer 118 controlled by patient 100 (*e.g.,* in their home, or in their relative's home) and/or smart watch 120 worn by health care personnel 106 (or by an organism), to render GUIs configured with selected aspects of the present disclosure.

In some implementations, UI engine 116 may distribute data in various forms to cause GUIs configured with selected aspects of the present disclosure to be rendered on remote computing devices. In some implementations, UI engine 116 may distribute markup language documents (*e.g.,* HTML, XML) that are rendered by web browsers or other applications. In some embodiments, UI engine 116 may distribute graphics data that is usable at remote computing devices to render continuous physiological flow graphical elements and/or the visually-annotated quadrants they pass through in accordance with selected aspects of the present disclosure. In various implementations, these graphics data may take the form of vector graphics, rasterized/bitmap graphics, *etc.* In other implementations, UI engine 116 may be implemented in whole or in part on a client device, *e.g.,* based on data received at the client device from UI engine 116.

In some implementations, UI engine 116 may obtain measured physiological parameters from other information systems (*e.g.,* 112, 114), from laboratories, and/or from equipment being used to monitor physiological parameter(s) of patient 100, normalize and analyze these data points, and based on the analysis, render graphical elements that convey various aspects of the oxygenation process system of patient 100. While depicted separately from HIS 112 and EMR/PDMS 114 in Fig. 1, in other embodiments, UI engine 116 may be integral with either of these other information systems. In some embodiments, patient 100 may also wear a smart watch and/or wearable patient monitor (not depicted) that includes a display on which GUIs configured with aspects of the present disclosure may be rendered.

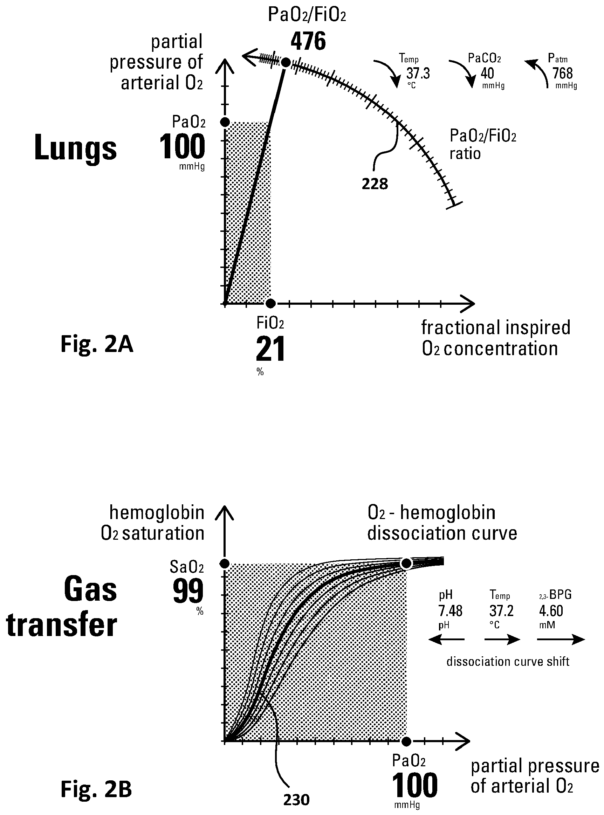

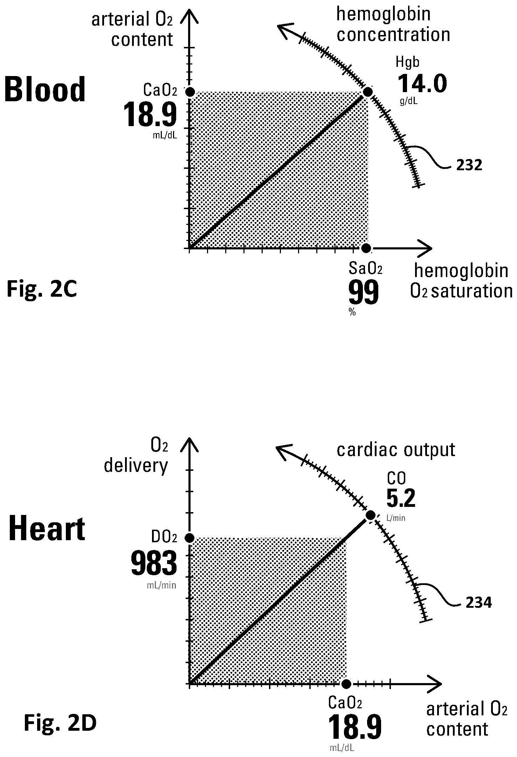

Figs. 2A, 2B, 2C, and 2D depict examples of how individual quadrants of a GUI rendered in accordance with aspects of the present disclosure may correspond to various aspects and/or stages of an oxygenation process, in accordance with selected aspects of the present disclosure. In Fig. 2A, a first graph corresponding to a first quadrant is labeled as "Lungs" because the data of the graph represents transfer of oxygen from inhaled air into blood of patient 100. In particular, an abscissa (horizontal axis in Fig. 2A) corresponds to "fraction of inspired oxygen" or "fractional O2 concentration" (FiO₂) and an ordinate (vertical axis in Fig. 2A) corresponds to partial pressure of arterial oxygen (PaO₂).

Thus, the data plotted on the graph of Fig. 2A represents the FiO₂/PaO₂ ratio, which is an indication of the effectiveness of the patient's inhalation of oxygen (whether normal atmospheric oxygen or supplemental oxygen). The higher the ratio, the more efficient the transfer of oxygen from the lungs of patient 100 to blood. In Fig. 2A, for instance, the FiO₂/PaO₂ ratio is 476. This ratio may be influenced by a variety of factors, some of which are conveyed by visual annotations at the top right, such as a temperature of patient 100, a partial pressure of carbon dioxide (PaCO₂) of patient 100, a measure of atmospheric pressure (Patm) in the environment of patient 100, *etc.* Greater measures of the former two factors may reduce the FiO₂/PaO₂ ratio, whereas greater measure of the latter factor may increase the FiO₂/PaO₂ ratio. In Fig. 2A, the current FiO₂/PaO₂ ratio is plotted on a separate FiO₂/PaO₂ ratio curve 228 of the GUI. FiO₂/PaO₂ ratio curve 228 visually conveys to a user the possible values of the FiO₂/PaO₂ ratio.

As indicated by the "Gas transfer" label, the graph depicted in Fig. 2B represents a relationship between PaO₂ as the abscissa and saturation of blood hemoglobin with oxygen (SaO₂) as the ordinate. In some embodiments, the relationship between these physiological parameters may be governed and represented visually by an O₂-hemoglobin dissociation curve 230. O₂-hemoglobin dissociation curve 230 plots the proportion of hemoglobin in its saturated form as the ordinate against the prevailing oxygen tension as the abscissa. The position of a data point on O₂-hemoglobin dissociation curve 230 may indicate a slope of a tangent line, which is an important aspect of situational evaluation of patient 100.

Similar to Fig. 2A, various factors are visually indicated at right in Fig. 2B to convey how they influence shifts of O₂-hemoglobin dissociation curve 230. These factors include blood pH of patient 100, temperature of patient 100, and a level of 2,3-Bisphosphoglyceric acid (_{2,3}-BPG) of patient 100. Arrows are rendered underneath these factors in order to demonstrate to the user how those factors (*i.e.* their current values) are impacting O₂-hemoglobin dissociation curve 230. For example, the pH value of 7.48 shifts O₂-hemoglobin dissociation curve 230 left, whereas the 4.56 value of _{2,3}-BPG shifts O₂-hemoglobin dissociation curve 230 right.

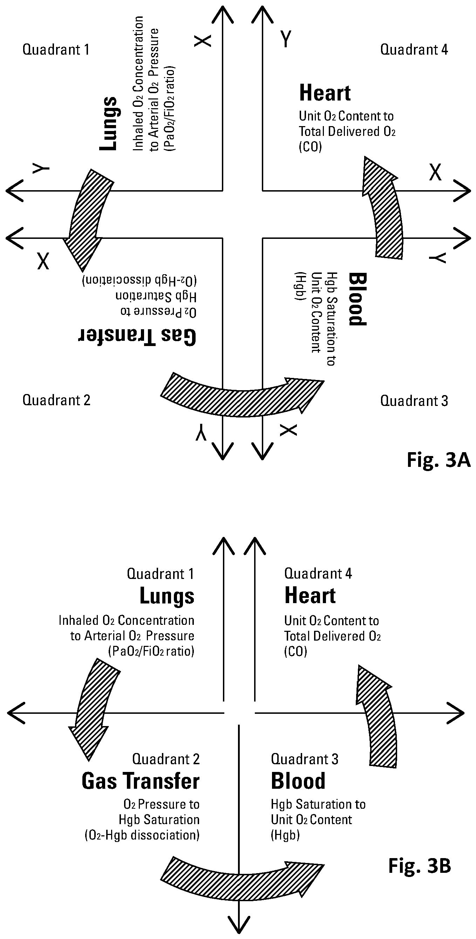

Fig. 2C is labeled "Blood" to indicate an important factor in the effectiveness of oxygenation, namely, the amount of hemoglobin (Hgb) in the blood of patient 100. In Fig. 3, the abscissa corresponds to SaO₂, and the ordinate corresponds to arterial oxygen content (CaO₂). A separate Hgb curve 232 shows the potential range of Hgb values, with the current Hgb value being 14.0 g/dL. When hemoglobin content is low, even 100% oxygen saturation results in low oxygen content per unit of blood volume.

Fig. 2D is labeled "Heart" to indicate that it deals with an impact of cardiac output (CO) of patient 100 on an amount of oxygen delivered to tissues (DO₂) of patient 100. The abscissa of the graph in Fig. 2D is CaO₂ and the ordinate is DO₂. Also rendered is a separate CO curve 234 that shows potential values of CO of patient 100 (currently 5.2 L/min). This graph demonstrates the simple notion that the greater the blood flow, the more oxygen is delivered to tissues of patient 100.

Figs. 3A and 3B demonstrate examples of how the quadrants of Figs. 2A-D may be arranged visually relative to one another in accordance with various embodiments. These figures particularly demonstrate how in various embodiments, a GUI rendered on a display in accordance with the present disclosure may arrange the quadrants shown in Figs. 2A-D so that for at least some of the four quadrants, the ordinate of one quadrant corresponds to the abscissa of the next quadrant of the cycle.

The direction of flow between the four quadrants in Figs. 3A and 3B is counterclockwise, as indicated by the arrows, but this is not meant to be limiting. In various embodiments, the flow may be clockwise instead. Moreover, while the flow starts at top left in the figures herein, in other embodiments, the quadrant labeled "Lungs" may be positioned elsewhere in relation to the other quadrants. As used herein, the terms "downstream" and "upstream" are used relative to the direction of flow. Thus, the second quadrant ("Gas transfer") is downstream from the first quadrant ("Lungs"), the third quadrant ("Blood") is downstream from the second quadrant ("Gas transfer"), the first quadrant is upstream from the second quadrant, and so on.

In Fig. 3A, the first quadrant entitled "Lungs" is situated at top left and corresponds to the graph depicted in Fig. 2A. In various embodiments, the abscissa of the first "Lungs" quadrant may correspond to FiO₂ of patient 100 and the ordinate may correspond to PaO₂ of patient 100. Additionally, the ordinate (PaO₂) of the first "Lungs" quadrant may correspond to the abscissa of the second quadrant entitled "Gas Transfer" at bottom left. In this second "Gas Transfer" quadrant, which corresponds to the graph depicted in Fig. 2B, the ordinate corresponds to SaO₂ of patient 100.

The ordinate (SaO₂) of the second "Gas Transfer" quadrant at bottom left also may correspond to the abscissa of the third quadrant entitled "Blood" at bottom right. In this third "Blood" quadrant, which corresponds to the graph depicted in Fig. 2C, the ordinate corresponds to CaO₂. Additionally, the ordinate (CaO₂) of the third "Blood" quadrant at bottom right may correspond to the abscissa of the fourth quadrant entitled "Heart" at top right, which corresponds to the graph depicted in Fig. 2D.

Fig. 3B depicts fully integrated compound graph, where the ordinate of one quadrant merges with the abscissa of the quadrant downstream from it, thereby representing continuous connection between the various depicted physiological measures of "Lungs", "Gas Transfer", "Blood", and "Heart" sections.

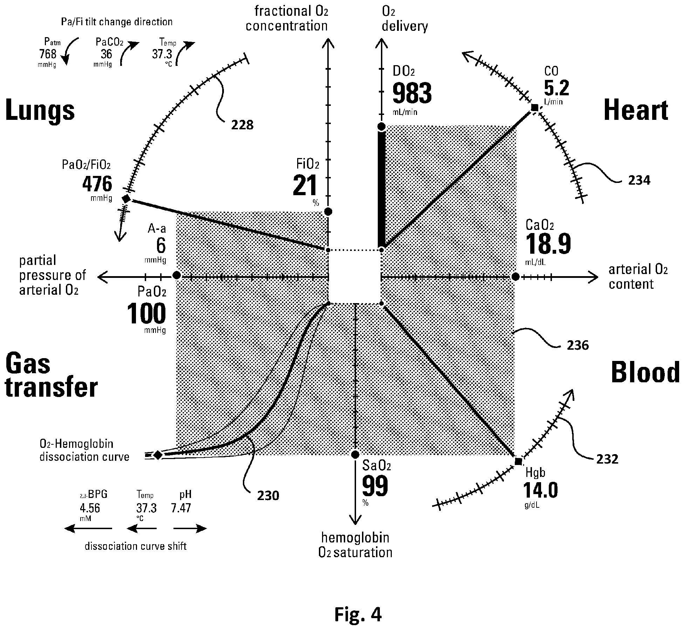

In various implementations, the graphs shown in Figs. 2A-D can be arranged as indicated by Fig. 3B in a manner that facilitates quick understanding by clinicians. Fig. 4 depicts one example of this in an example GUI configured with selected aspects of the present disclosure. In Fig. 4, a continuous physiological flow graphical element 236 is shown that passes through each of the four quadrants in a counterclockwise direction starting in the top left quadrant at physiological parameter FiO₂. This presents, as part of the GUI, a visual overview of the patient's oxygen delivery system.

A vertical thickness of continuous physiological flow graphical element 236 in the first quadrant ("Lungs") corresponds to a value of the abscissa in the first quadrant, namely, the FiO₂ of patient 100, which is currently 21%. The PaO₂/FiO₂ ratio of patient 100 is currently 476 mmHg (100/0.21). The angle of the line representing this ratio in turn dictates the width (horizontal thickness) of the continuous physiological flow graphical element 236 in this first quadrant, which corresponds to the value of PaO2 of patient 100, which is currently 100 mmHg. Also rendered at top left in Fig. 4 are graphical elements that convey values of the other factors (*e.g*., Patm, PaCO₂, Temp.) that can impact the PaO₂/FiO₂ ratio, as well as the additional curve 228 that demonstrates where the current PaO₂/FiO₂ ratio falls amongst potential PaO₂/FiO₂ ratios. Also included in the first quadrant of the GUI, as additional numerical data, is the alveolar to arterial (A-a) oxygen gradient of 6 mmHg. In various embodiments, these additional graphical elements (and elsewhere in general) may or may not be rendered as part of the GUI, *e.g.,* depending on preferences of the user, preferences of an administrator, and so forth.

The horizontal thickness of continuous physiological flow graphical element 236 in the first quadrant ("Lungs"), which is currently 100 mmHg, also corresponds to a horizontal distance in the second quadrant ("Gas transfer") between the PaO₂ value (100 mmHg) and a top tip of O₂-hemoglobin dissociative curve 232. Put another way, a width of continuous physiological flow graphical element 236 that passes from the second quadrant to the third quadrant is dictated by an intersection of continuous physiological flow graphical element 236 with oxygen-hemoglobin dissociation curve 230. Also rendered as part of GUI are other factors that influence O₂-hemoglobin dissociative curve 232, such as _{2,3}.BPG, Temp, and blood ph.

A vertical thickness of continuous physiological flow graphical element 236 in the second quadrant ("Gas transfer") corresponds to a value of the ordinate of the second quadrant, namely, SaO₂. (with a current value of 99%). This vertical thickness also corresponds to the value of the abscissa in the third quadrant ("Blood") at bottom right, also SaO₂. In the third quadrant ("Blood"), a horizontal thickness of continuous physiological flow graphical element 236 corresponds to the current ordinate value, *i.e.* a CaO₂ of 18.9 mL/dL. The curve 232 is also rendered in the third quadrant ("Blood") to show the current and potential values of Hgb, which currently stands at 14.0 g/dL.

In the fourth quadrant ("Heart"), a vertical thickness of continuous physiological flow graphical element 236 corresponds to a current rate of oxygen delivery (DO₂) to tissues of patient 100, which current stands at 983 mL/min. As mentioned previously, the curve 234 in the fourth quadrant ("Heart") shows the current and potential CO values. Depending on a current value of CO, a particular CaO₂ yields a particular amount of DO₂.

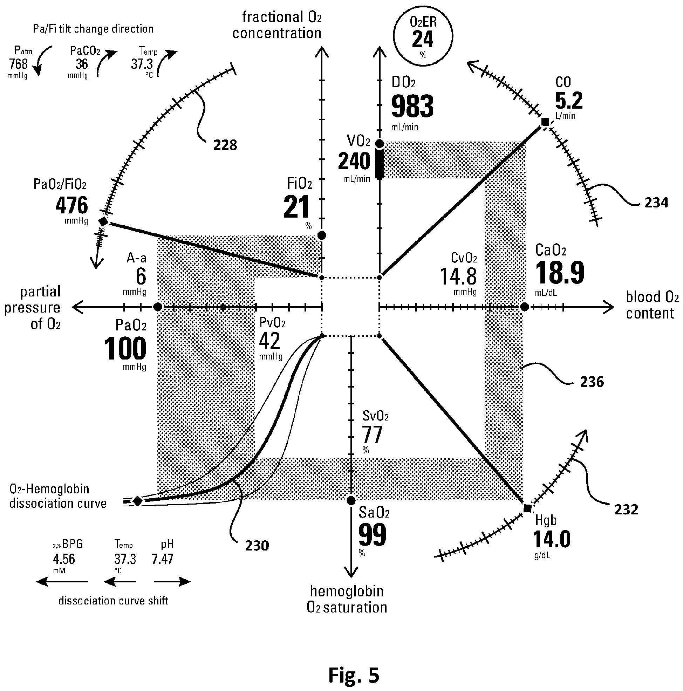

Fig. 5 depicts another example GUI configured with selected aspects of the present disclosure that conveys oxygenation parameters of a healthy patient. In this example, information about oxygenation of venous blood is available and is used to supplement the aspects of the GUI depicted in Fig. 4. In particular, it is possible with this additional data to convey an extent of reserve oxygen in the patient's body. One additional physiological parameter is oxygen consumption (VO₂, currently 240 mL/min), which is conveyed via a visual annotation in between the first quadrant ("Lungs") and the fourth quadrant ("Heart"). Another additional physiological parameter is oxygen extraction ratio (O₂ER), which is conveyed via a visual annotation in the fourth quadrant ("Heart") with a current value of 24%. Conveyed together, these two additional physiological parameters provide an indication of strain undergone by a patient's circulatory system while it fulfills the patient's current O₂ utilization needs.

While not shown in Fig. 5, in other embodiments, visual annotations of other parameters associated with ventilation may also be provided in some embodiments, similar to the temperatures, PaCO₂ and Patm described elsewhere. Moreover, and also depicted in Fig. 5 are visual annotations of other parameters, including mixed venous oxygen tension (PvO₂), mixed venous oxygen saturation (SvO₂), and mixed venous oxygen content (CvO₂). These other parameters may influence a size and/or shape of continuous physiological flow graphical element 236, as shown in Fig. 5.

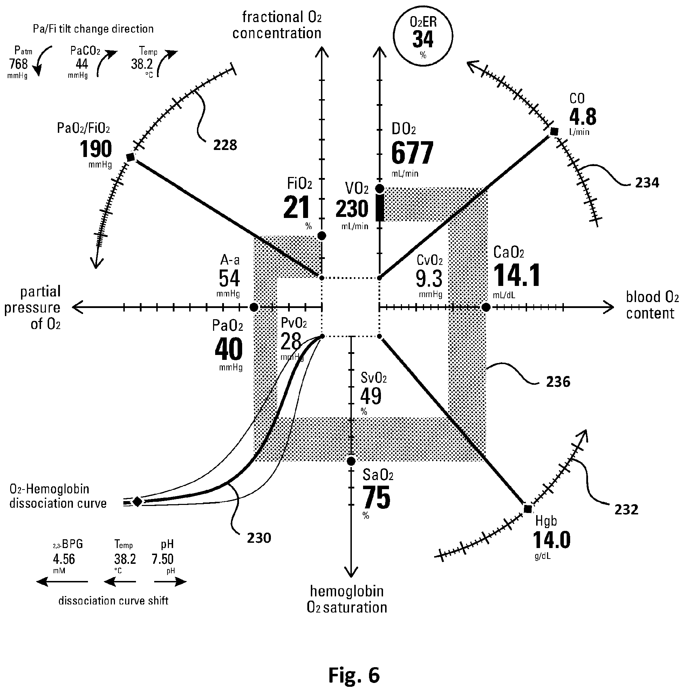

Unlike Fig. 5, which depicts oxygenation parameters of a healthy patient, Figs. 6-8 depict a GUI configured with selected aspects of the present disclosure for unhealthy patients. Fig. 6 shows how deteriorated lung function causes PaO₂ (ordinate of the first quadrant at top left, abscissa of the second quadrant at bottom left) to drop. This causes the O₂-Hgb dissociation value to be pushed into a steep part of O₂-Hgb dissociation curve 230, where a small decrease in O₂ leads to a sharp decrease in hemoglobin saturation. Despite relatively high hemoglobin content and healthy cardiac output, DO₂ is dangerously low and O₂ER is outside of normal range. The example of Fig. 6 shows that the problem is related to the second, "gas transfer" quadrant, and that the patient is undergoing acute respiratory distress syndrome (ARDS).

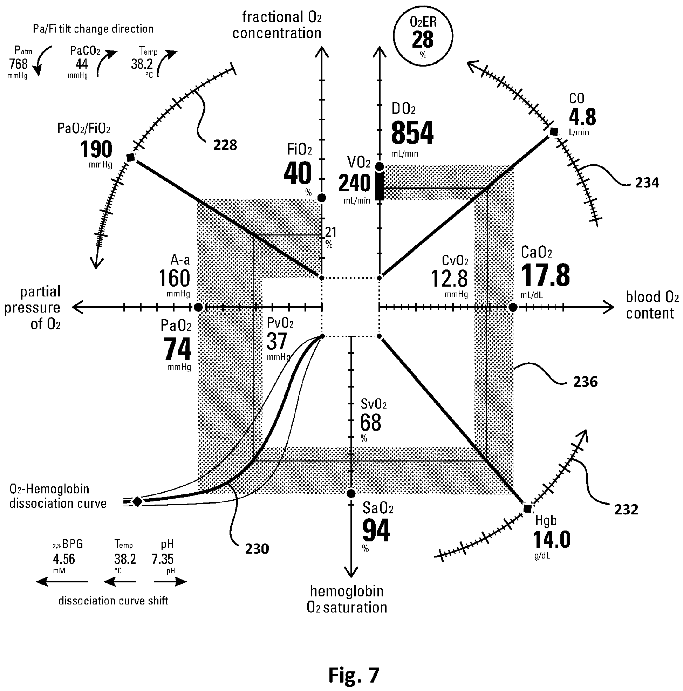

Fig. 7 shows how continuous physiological flow graphical element 236 may appear for a patient in a similar condition as the patient of Fig. 6, except that the patient is treated with supplemental oxygen. Though the lung function (PaO₂/FiO₂ ratio) still is suggestive of at least moderate ARDS, the increased FiO₂ has correspondingly increased DO₂ and O₂ER into borderline acceptable ranges. Notably, overlaying a portion of continuous physiological flow graphical element 236 in the first quadrant ("Lungs"), a thin line has been rendered at 21% of FiO₂. This thin line may represent a threshold below which various oxygenation parameters would fall without supplemental O₂. In other words, the patient has been stabilized by the current FiO₂ value of 40%, but should not be allowed to fall under 21%.

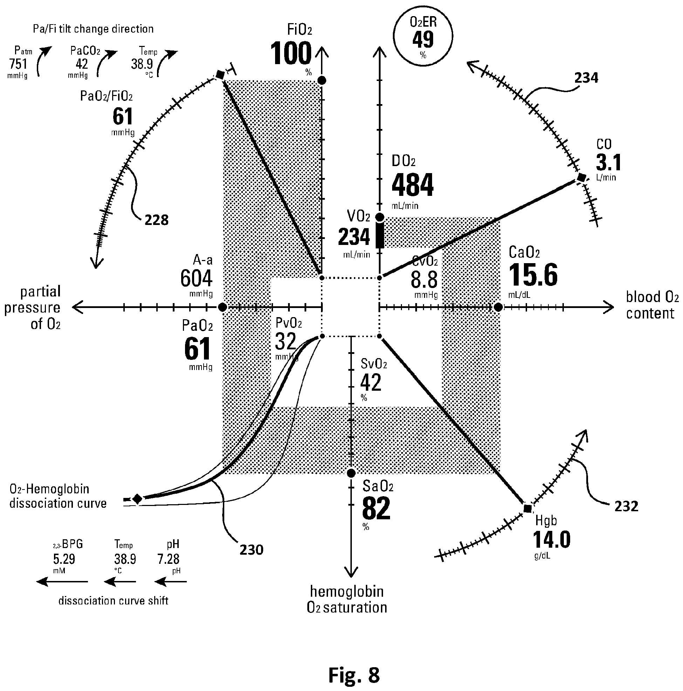

Fig. 8 shows how continuous physiological flow graphical element 236 may appear for a patient with severely compromised lungs, *e.g.,* who is receiving 100% supplemental oxygen. With PaO₂/FiO₂ ratio of 61 this patient would be classified as being in severe ARDS. Because of heightened temperature and low pH, O₂-hemoglobin dissociation curve 230 has shifted, causing further deterioration in SaO₂. Though the hemoglobin content is healthy, low saturation combined with weak CO result in low DO₂ and dangerously high O₂ER of 49%.

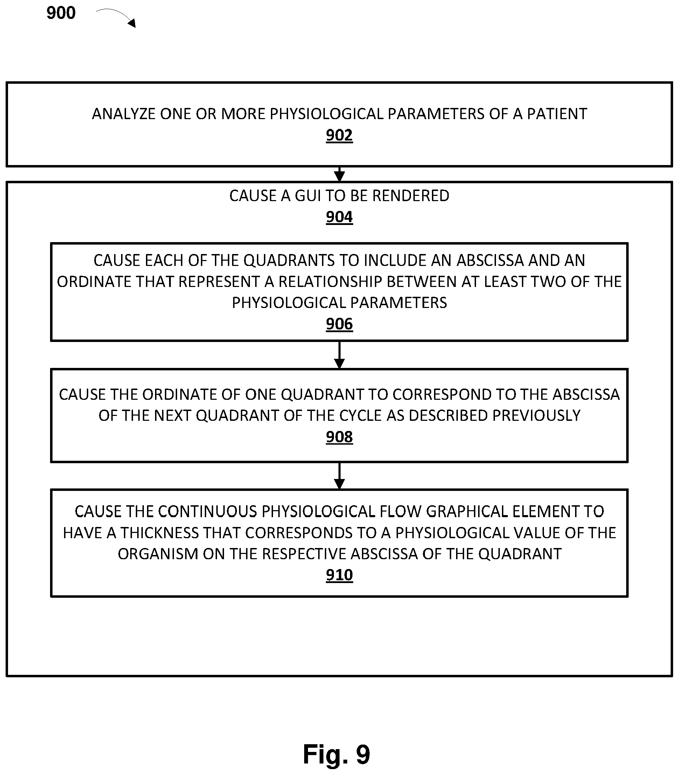

Referring now to Fig. 9, an example method 900 of practicing selected aspects of the present disclosure is described. For convenience, the operations of the flowchart are described with reference to a system that performs the operations. This system may include various components of various computer systems. For instance, various operations may be performed by one or more components of UI engine 116 or other components of Fig. 1. Moreover, while operations of method 900 are shown in a particular order, this is not meant to be limiting. One or more operations may be reordered, omitted or added.

At block 902, the system may analyze one or more physiological parameters of an organism such as patient 100. Based on the analyzing, at block 904, the system may cause a GUI to be rendered on a display. In various implementations, the GUI conveys oxygenation of an organism visually as a continuous physiological flow graphical element 236 that passes through four quadrants in a cycle. For example, at block 906, the system may cause each of the quadrants to include an abscissa and an ordinate that represent a relationship between at least two of the physiological parameters. At block 908, for at least some of the four quadrants, the system may cause the ordinate of one quadrant to correspond to the abscissa of the next quadrant of the cycle as described previously. At block 910, the system may, for each of the four quadrants, cause the continuous physiological flow graphical element to have a thickness that corresponds to a physiological value of the organism on the respective abscissa of the quadrant.

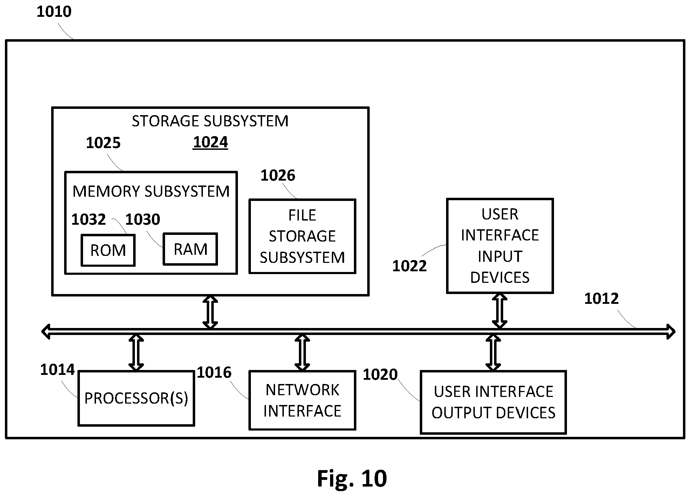

Fig. 10 is a block diagram of an example computing device 1010 that may optionally be utilized to perform one or more aspects of techniques described herein. Computing device 1010 typically includes at least one processor 1014 which communicates with a number of peripheral devices via bus subsystem 1012. These peripheral devices may include a storage subsystem 1024, including, for example, a memory subsystem 1025 and a file storage subsystem 1026, user interface output devices 1020, user interface input devices 1022, and a network interface subsystem 1016. The input and output devices allow user interaction with computing device 1010. Network interface subsystem 1016 provides an interface to outside networks and is coupled to corresponding interface devices in other computing devices.

User interface input devices 1022 may include a keyboard, pointing devices such as a mouse, trackball, touchpad, or graphics tablet, a scanner, a touchscreen incorporated into the display, audio input devices such as voice recognition systems, microphones, and/or other types of input devices. In general, use of the term "input device" is intended to include all possible types of devices and ways to input information into computing device 1010 or onto a communication network.

User interface output devices 1020 may include a display subsystem, a printer, a fax machine, or non-visual displays such as audio output devices. The display subsystem may include a cathode ray tube (CRT), a flat-panel device such as a liquid crystal display (LCD), a projection device, or some other mechanism for creating a visible image. The display subsystem may also provide non-visual display such as via audio output devices. In general, use of the term "output device" is intended to include all possible types of devices and ways to output information from computing device 1010 to the user or to another machine or computing device.

Storage subsystem 1024 stores programming and data constructs that provide the functionality of some or all of the modules described herein. For example, the storage subsystem 1024 may include the logic to perform selected aspects of the method of Fig. 9, as well as to implement various components depicted in Fig. 1.

These software modules are generally executed by processor 1014 alone or in combination with other processors. Memory 1025 used in the storage subsystem 1024 can include a number of memories including a main random access memory (RAM) 1030 for storage of instructions and data during program execution and a read only memory (ROM) 1032 in which fixed instructions are stored. A file storage subsystem 1026 can provide persistent storage for program and data files, and may include a hard disk drive, a floppy disk drive along with associated removable media, a CD-ROM drive, an optical drive, or removable media cartridges. The modules implementing the functionality of certain implementations may be stored by file storage subsystem 1026 in the storage subsystem 1024, or in other machines accessible by the processor(s) 1014.

Bus subsystem 1012 provides a mechanism for letting the various components and subsystems of computing device 1010 communicate with each other as intended. Although bus subsystem 1012 is shown schematically as a single bus, alternative implementations of the bus subsystem may use multiple busses.

Computing device 1010 can be of varying types including a workstation, server, computing cluster, blade server, server farm, or any other data processing system or computing device. Due to the ever-changing nature of computers and networks, the description of computing device 1010 depicted in Fig. 10 is intended only as a specific example for purposes of illustrating some implementations. Many other configurations of computing device 1010 are possible having more or fewer components than the computing device depicted in Fig. 10.

While several inventive embodiments have been described and illustrated herein, those of ordinary skill in the art will readily envision a variety of other means and/or structures for performing the function and/or obtaining the results and/or one or more of the advantages described herein, and each of such variations and/or modifications is deemed to be within the scope of the inventive embodiments described herein. More generally, those skilled in the art will readily appreciate that all parameters, dimensions, materials, and configurations described herein are meant to be exemplary and that the actual parameters, dimensions, materials, and/or configurations will depend upon the specific application or applications for which the inventive teachings is/are used. Those skilled in the art will recognize, or be able to ascertain using no more than routine experimentation, many equivalents to the specific inventive embodiments described herein. It is, therefore, to be understood that the foregoing embodiments are presented by way of example only and that, within the scope of the appended claims and equivalents thereto, inventive embodiments may be practiced otherwise than as specifically described and claimed. Inventive embodiments of the present disclosure are directed to each individual feature, system, article, material, kit, and/or method described herein. In addition, any combination of two or more such features, systems, articles, materials, kits, and/or methods, if such features, systems, articles, materials, kits, and/or methods are not mutually inconsistent, is included within the inventive scope of the present disclosure.

All definitions, as defined and used herein, should be understood to control over dictionary definitions, definitions in documents incorporated by reference, and/or ordinary meanings of the defined terms.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, *i.e*., elements that are conjunctively present in some cases and disjunctively present in other cases. Multiple elements listed with "and/or" should be construed in the same fashion, *i.e*., "one or more" of the elements so conjoined. Other elements may optionally be present other than the elements specifically identified by the "and/or" clause, whether related or unrelated to those elements specifically identified. Thus, as a non-limiting example, a reference to "A and/or B", when used in conjunction with open-ended language such as "comprising" can refer, in one embodiment, to A only (optionally including elements other than B); in another embodiment, to B only (optionally including elements other than A); in yet another embodiment, to both A and B (optionally including other elements); *etc.*

As used herein in the specification and in the claims, "or" should be understood to have the same meaning as "and/or" as defined above. For example, when separating items in a list, "or" or "and/or" shall be interpreted as being inclusive, *i.e*., the inclusion of at least one, but also including more than one, of a number or list of elements, and, optionally, additional unlisted items. Only terms clearly indicated to the contrary, such as "only one of' or "exactly one of," or, when used in the claims, "consisting of," will refer to the inclusion of exactly one element of a number or list of elements. In general, the term "or" as used herein shall only be interpreted as indicating exclusive alternatives (*i.e.* "one or the other but not both") when preceded by terms of exclusivity, such as "either," "one of," "only one of," or "exactly one of." "Consisting essentially of," when used in the claims, shall have its ordinary meaning as used in the field of patent law.

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified. Thus, as a non-limiting example, "at least one of A and B" (or, equivalently, "at least one of A or B," or, equivalently "at least one of A and/or B") can refer, in one embodiment, to at least one, optionally including more than one, A, with no B present (and optionally including elements other than B); in another embodiment, to at least one, optionally including more than one, B, with no A present (and optionally including elements other than A); in yet another embodiment, to at least one, optionally including more than one, A, and at least one, optionally including more than one, B (and optionally including other elements); *etc.*

It should also be understood that, unless clearly indicated to the contrary, in any methods claimed herein that include more than one step or act, the order of the steps or acts of the method is not necessarily limited to the order in which the steps or acts of the method are recited.

In the claims, as well as in the specification above, all transitional phrases such as "comprising," "including," "carrying," "having," "containing," "involving," "holding," "composed of," and the like are to be understood to be open-ended, *i.e*., to mean including but not limited to. Only the transitional phrases "consisting of' and "consisting essentially of' shall be closed or semi-closed transitional phrases, respectively, as set forth in the United States Patent Office Manual of Patent Examining Procedures, Section 2111.03. It should be understood that certain expressions and reference signs used in the claims pursuant to Rule 6.2(b) of the Patent Cooperation Treaty ("PCT") do not limit the scope.

## Claims

1. A method implemented using one or more processors, comprising:
analyzing (902) one or more physiological parameters of an organism;
based on the analyzing, causing (904) a graphical user interface (GUI) to be rendered on a display, wherein the GUI conveys oxygenation of an organism visually as a continuous physiological flow graphical element (236) that passes through four quadrants in a cycle, wherein the causing includes:
causing (906) each of the quadrants to include an abscissa and an ordinate that represent a relationship between at least two of the physiological parameters; and
for at least some of the four quadrants, causing (908) the ordinate of one quadrant to correspond to the abscissa of the next quadrant of the cycle.

2. The method of claim 1, wherein in a first quadrant of the four quadrants that conveys information about lung function in the organism, the abscissa corresponds to fractional oxygen concentration (FiO₂) of the organism and the ordinate corresponds to partial pressure of arterial oxygen (PaO₂) of the organism.

3. The method of claim 2, wherein in a second quadrant of the four quadrants that conveys information about gas transfer in the organism, the abscissa corresponds to PaO₂ of the organism and the ordinate corresponds to hemoglobin oxygen saturation (SaO₂) of the organism.

4. The method of claim 3, comprising visually conveying the relationship between PaO₂ values of the abscissa and SaO₂ value of the ordinate by causing an oxygen-hemoglobin dissociation curve (230) to be rendered in the second quadrant.

5. The method of claim 3, comprising rendering one or more additional visual annotations that convey one or more additional parameters that influence a portion of the oxygen-hemoglobin dissociation curve.

6. The method of claim 5, wherein in a third quadrant of the four quadrants that conveys information about blood of the organism, the abscissa corresponds to SaO₂ of the organism and the ordinate corresponds to arterial oxygen content (CaO₂) of the organism.

7. The method of claim 6, wherein a width of the continuous physiological flow graphical element that passes from the second quadrant to the third quadrant is dictated by an intersection of the continuous physiological flow graphical element with the oxygen-hemoglobin dissociation curve.

8. The method of claim 6, wherein in a fourth quadrant of the four quadrants that conveys information about cardiac output of the organism, the abscissa corresponds to CaO₂ of the organism and the ordinate corresponds to oxygen delivery (DO₂) of the organism.

9. The method of claim 1, comprising, for each of the four quadrants, causing the continuous physiological flow graphical element to have a thickness that corresponds to a physiological value of the organism on the respective abscissa of the quadrant.

10. A system comprising one or more processors and memory storing instructions that, in response to execution of the instructions by the one or more processors, cause the one or more processors to:
analyze (902) one or more physiological parameters of an organism;
based on the analysis, cause (906) a graphical user interface (GUI) to be rendered on a display, wherein the GUI conveys oxygenation of an organism visually as a continuous physiological flow graphical element that passes through four quadrants in a cycle, wherein:
each of the quadrants includes an abscissa and an ordinate that represent a relationship between at least two of the physiological parameters; and
for at least some of the four quadrants, the ordinate of one quadrant corresponds to the abscissa of the next quadrant of the cycle.

11. The system of claim 10, wherein in a first quadrant of the four quadrants that conveys information about lung function in the organism, the abscissa corresponds to fractional oxygen concentration (FiO₂) of the organism and the ordinate corresponds to partial pressure of arterial oxygen (PaO₂) of the organism.

12. The system of claim 11, wherein in a second quadrant of the four quadrants that conveys information about gas transfer in the organism, the abscissa corresponds to PaO₂ of the organism and the ordinate corresponds to hemoglobin oxygen saturation (SaO₂) of the organism.

13. The system of claim 12, comprising instructions to visually convey the relationship between PaO₂ values of the abscissa and SaO₂ value of the ordinate by causing an oxygen-hemoglobin dissociation curve (230) to be rendered in the second quadrant.

14. The system of claim 13, wherein in a third quadrant of the four quadrants that conveys information about blood of the organism, the abscissa corresponds to SaO₂ of the organism and the ordinate corresponds to arterial oxygen content (CaO₂) of the organism.

15. At least one non-transitory computer-readable medium comprising instructions that, in response to execution of the instructions by one or more processors, cause the one or more processors to perform the method of any one of claims 1-9.
